# EUROPEAN PATENT SPECIFICATION

(11) **EP 0 900 428 B1**
(45) Date of publication and mention of the grant of the patent: **31.08.2005**
(21) Application number: 97950706.8
(22) Date of filing: 26.11.1997
(51) Int. Cl.: G06T 5/20

(54) **MULTIPLE MODE DIGITAL X-RAY IMAGING SYSTEM**
VIELFACHMODUS RÖNTGENSTRAHLUNGS-BILDAUFNAHMESYSTEM
SYSTEME D'IMAGERIE NUMERIQUE AUX RAYONS X A MODES MULTIPLES

(30) Priority: 29.11.1996 US 753799; 29.11.1996 US 56926 P; 25.11.1997 US 978177
(43) Date of publication of application: 10.03.1999
(60) Divisional application: 03075481.6
(73) Proprietor: Varian Medical Systems Technologies, Inc., Palo Alto, CA 94304-1038 (US)
(72) Inventor: COLBETH, Richard, E., Los Altos, CA 94024 (US); PAVOKOVICH, John, M., Palo Alto, CA 94304 (US); SEPPI, Edward, J., Portola Valley, CA 94028 (US); SHAPIRO, Edward, G., Mountain View, CA 94043 (US)
(74) Representative: Foster, Mark Charles
(86) International application number: PCT/US1997/021685
(87) International publication number: WO 1998/024059

(56) References cited:
- EP-A- 0 776 124
- US-A- 5 657 400

## Description

### BACKGROUND OF THE INVENTION

The present invention relates to radiation imaging systems, and in particular, to solid state X-ray radiation imaging systems capable of operating in multiple detection and display modes.

### DESCRIPTION OF THE RELATED ART

The use of X-ray radiation has become a valuable and widespread tool in medical diagnoses and treatments. In film radiography, a burst of X-rays, after passing through the body, is recorded on high resolution X-ray film. In fluoroscopy, an image intensifier tube converts X-ray radiation to a video signal for viewing and recording interior body activity as a video image.

Film radiography is commonly used due to its good spatial resolution, high signal-to-noise ratio (SNR), large detection area and low cost. However, developing exposed X-ray film typically takes a minimum of ninety seconds which can be too long in emergency situations. Further, the relatively low dynamic range of X-ray film can result in under- or over-exposed images and, therefore, necessitate additional exposures which increase the aforementioned time delay as well as the X-ray dosage received by the patient.

The image intensifier tube used in fluoroscopy has a greater exposure latitude than X-ray film, but also has a more limited active detection area and lower spatial resolution. The lower spatial resolution associated with the total active area is somewhat mitigated in that the image intensifier tubes allow magnification of the central image portion, thereby providing a means to enhance visual details. However, the image intensifier tube is typically heavy, bulky and expensive, and can introduce image distortion which can only be partially removed during post processing.

A number of alternative X-ray imaging technologies have been developed. For example, one alternative, known as computed radiography, involves the use of a photostimulable phosphor plate which has the same physical appearance as a standard X-ray film cassette and provides good spatial resolution. SNR and dynamic range. However, after exposure to X-rays, the photostimulable phosphor plate must be scanned with a laser system which is large and expensive, and the readout process is just as slow as the development of film.

Another alternative which provides good spatial resolution and dynamic range, as well as the added advantage of compatibility with real time digital image processing techniques, involves the use of solid state detector panels. One such panel uses an amorphous silicon (a-Si) detector array arranged as a two dimensional matrix of pixels, each of which consists of a photosensitive element and a transistor switch. As with X-ray film cassettes, the detector array is covered with a scintillation layer to convert impinging X-rays into visible light for the photosensitive elements.

EP-A-0776124 discloses an arrangement for imaging an object by detecting radiation reflected from and/or transmitted through the object using an imaging sensor system. To perform an optimised selection between the imaging resolution and the sensitivity of the imaging sensor, the sensor system is configured by means of a control scheme based on combining, or binning, the radiation-responsive imaging elements of the image sensor along the rows and/or columns of the sensor matrix into clusters of a plurality of imaging elements.

### SUMMARY OF THE INVENTION

An X-ray imaging system in accordance with the present invention may be capable of operating in multiple imaging modes, such as radiographic and fluoroscopic, while providing spatial resolutions, SNRs and dynamic ranges which can be selectively optimised to the selected mode of operation.

The present invention is defined in the independent claims.

In accordance with an embodiment of the present invention, the combining of a pixel information collected by the detector array, i.e., "pixel binning," is performed by selectively combining one portion of the pixel information within the detector array and selectively combining the remainder of the pixel information within the circuits fed by the output of the detector array. Such pixel binning is preferably analog in nature and is performed prior to any digitizing of the pixel signals, thereby providing for a higher SNR, and, importantly, reducing the bandwidth requirements for the digital electronics. More specifically, a multiple mode X-ray detector system for supporting multiple X-ray image display modes by providing X-ray image signals having selectable spatial resolutions includes a detector array and a group of detector array receiver circuits. The detector array is configured to receive a group of detector control signals and in accordance therewith receive and convert X-ray photons corresponding to a two-dimensional image into a first group of image signals representing a first two-dimensional array which includes a first group of rows and a first group of columns of pixels which together correspond to the two-dimensional image and which individually correspond to respective portions of the two-dimensional image. The detector array provides, in accordance with the detector control signals, a second group of image signals representing a second two-dimensional array which includes a second group of rows and the first group of columns of super pixels which selectively represent respective individual ones or multiple adjacent ones of the first group of rows of pixels and respective individual ones of the first group of columns of pixels, respectively. The detector array receiver circuits, coupled to the detector array, are configured to receive a group of receiver control signals and in accordance therewith receive and combine the second group of image signals and in accordance therewith provide a third plurality of image signals representing a third two-dimensional array which includes the second group of rows and a second group of columns of super pixels which selectively represent respective individual ones of the second group of rows of super pixels and respective individual ones or multiple adjacent ones of the first group of columns of super pixels, respectively.

In accordance with an embodiment of the present invention, data flags are use to identify defective pixels within the detector array and are inserted into the data stream collected from the detector array for dynamic processing along with the pixel data. More specincally, a data processing system for processing a serial stream of multiple bit data sets which represent an array of pixels corresponding to a two-dimensional image including correcting for defective pixels individually or in groups includes a data processing circuit and a data selection circuit. The data processing circuit is configured to receive and process together a plurality of successive sets of image data with a corresponding plurality of successive sets of correction data and in accordance therewith provide a plurality of successive sets of corrected image data. The plurality of successive sets of image data represents a plurality of pixels corresponding to a two-dimensional image, the plurality of successive sets of correction data represents a plurality of correction factors, each one of the plurality of correction factors corresponds to a respective one of the plurality of pixels and each one of the plurality of successive sets of correction data includes a data subset which indicates whether the respective one of the plurality of pixels is defective. The data selection circuit, coupled to the data processing circuit, is configured to receive and select between individual ones of the plurality of successive sets of corrected image data and individual ones of the corresponding plurality of successive sets of correction data and in accordance therewith provide a plurality of successive sets of selected data. An individual one of the plurality of successive sets of selected data includes a corresponding individual one of the plurality of successive sets of correction data when the data subset indicates that the corresponding respective one of the plurality of pixels is defective, and the individual one of the plurality of successive sets of selected data includes a corresponding one of the plurality of successive sets of corrected image data when the data subset does not indicate that the corresponding respective one of the plurality of pixels is defective.

In accordance with an embodiment of the present invention; a data buffer and filter is used to perform still image capture during radiographic imaging and to recursively filter incoming image data during fluoroscopic imaging. More specifically, a digital data buffer and filter for selectively storing image pixel data, combining new incoming image pixel data with previously stored image pixel data and providing such combined image pixel data for display thereof in a still image mode or an image motion mode includes a data scaling and summing circuit and a data memory circuit. The data scaling and summing circuit is configured to receive and scale an input data signal, receive and scale a stored data sum signal and sum said scaled input data signal and said scaled stored data sum signal and in accordance therewith provide a data sum signal. The input data signal is scaled in accordance with a first scaling factor and the stored data sum signal is scaled in accordance with a second scaling factor. The input data signal includes a plurality of successive sets of image data, and each one of the plurality of successive sets of image data includes a plurality of pixel data with active and inactive data states and which corresponds to a two-dimensional image having a two-dimensional array including a plurality of rows and a plurality of columns of pixels which together correspond to the two-dimensional image and which individually correspond to respective portions of the two-dimensional image. The data memory circuit, coupled to the data scaling and summing circuit, is configured to receive and selectively store the data sum signal and provide the stored data sum signal. The data scaling and summing circuit and the data memory circuit cooperatively operate in one of a plurality of operational modes during reception of the plurality of successive sets of image data. In a first one of the plurality of operational modes (e.g., in fluoroscopic mode), the first scaling factor has a value which is between zero and unity, and the second scaling factor has a value which equals a difference between unity and the first scaling factor value. In a second one of the plurality of operational modes (e.g., in radiographic mode): the first scaling factor has a value which is initially unity when a first one of the plurality of successive sets of image data is in the inactive data state, remains unity when.a subsequent second one of the plurality of successive sets of image data is in the active data state and becomes zero when a further subsequent third one of the plurality of successive sets of image data is in the inactive data state; and the second scaling factor has a value which is initially zero, becomes unity when the subsequent second one of the plurality of successive sets of image data is in the active data state and remains unity thereafter.

These and other features and advantages of the present invention will be understood upon consideration of the following detailed description of the invention and the accompanying drawings.

### BRIEF DESCRIPTION OF THE DRAWINGS

For a better understanding of the present invention, embodiments will now be described by way of example, with reference to the accompanying drawings, in which:
Figure 1 is a functional block diagram of an X-ray imaging system in accordance with the present invention.
Figure 2 is an exploded perspective view of an X-ray detector cassette for an X-ray imaging system in accordance with the present invention.
Figure 3 is a schematic diagram of a portion of the detector array of Figure 2.
Figure 4 is a functional block diagram of the array driver circuit assemblies of Figure 2.
Figure 5 is a functional block diagram of the receiver circuit assembly of Figure 2.
Figure 6 is a functional block diagram of the readout circuits in the receiver circuit assembly of Figure 5.
Figure 7 is a simplified schematic diagram of several adjacent preamplifier circuits in the readout circuit of Figure 6.
Figure 8 is a functional block diagram of the portion of the computer and control system of Figure 1 in which the image data is processed for display in accordance with the selected mode of operation.
Figure 9 represents the format of the data correction instruction word used by the decode/select and pixel data averaging stages of Figure 8.
Figure 10 illustrates an example of how the grouping of defective pixels within the detector array determines the selection of north/south or east/west pixel data averaging.
Figure 11 is a functional block diagram of the pixel data averaging stages of Figure 8.
Figure 12 is a functional block diagram of the data buffer stage of Figure 8.
Figure 13 is a timing diagram illustrating the relative timing and values of the data scaling factors during a radiographic mode of operation.
Figure 14 is a functional block diagram of an alternative embodiment of the data buffer stage of Figure 8.

### DETAILED DESCRIPTION OF THE INVENTION

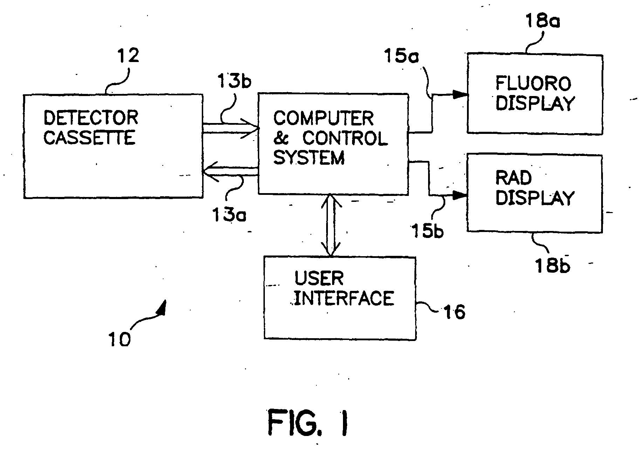

Referring to Figure 1, an X-ray imaging system 10 in accordance with the present invention includes a detector cassette 12, a computer and control system 14, a user interface 16, a fluoroscopic display 18a and a radiographic display 18b, interconnected substantially as shown. A user controls the system 10 by way of a user interface 16 (e.g., graphical user interface display, keyboard, mouse, etc.) which communicates with the computer and control system 14. Accordingly, the computer and control system 14 generates control signals 13a for the detector cassette 12 which provides image data signals 13b in return. (As desired, one display monitor could be used to selectively display both fluoroscopic and radiographic images, as well as the graphical user interface display image, e.g., all images could be displayed simultaneously in a "windowed" format, or either a fluoroscopic image or a radiographic image could be displayed along with a pull down menu bar, which menu bar constitutes the graphical user interface providing for selection of fluoroscopic or radiographic imaging.)

Following processing of such image data, the computer and control system 14 provides fluoroscopic image data 15a or radiographic image data 15b for display on a fluoroscopic display 18a or a radiographic display 18b, respectively, depending upon the selected mode of operation. The fluoroscopic display 18a preferably employs a phosphor which has a relatively short persistence time, thereby reducing unwanted ghost images when observing motion in the sequence of displayed images. The radiographic display 18b preferably employs a phosphor which yields a bluish tint to gray levels and has a relatively long persistence time, thereby replicating the bluish tint typically found in standard medical X-ray film images and reducing unwanted flicker in the displayed image.

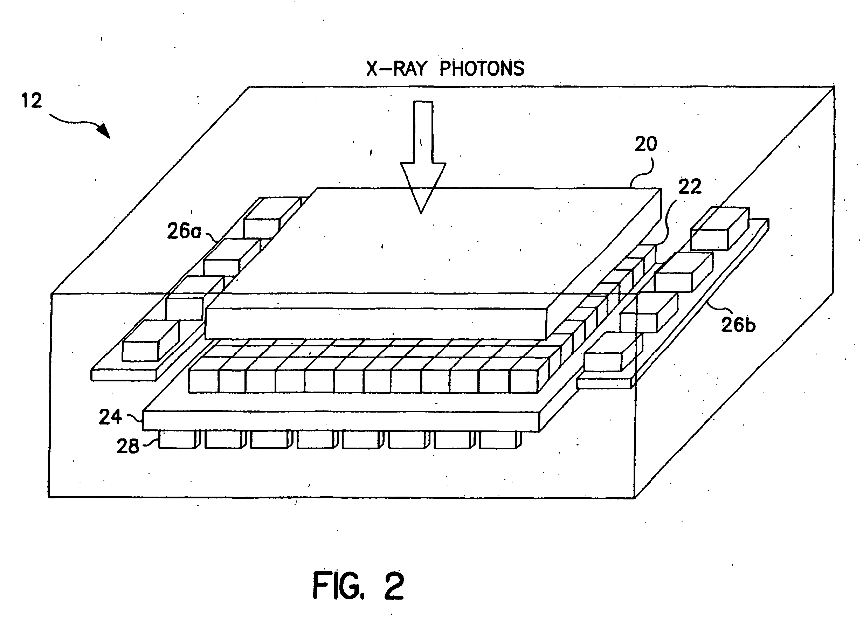

Referring to Figure 2, the detector cassette 12 is similar in external appearance to the typical cassette which contains standard medical X-ray film and is, therefore, highly mobile and easy to use as required for a radiographic mode of operation. A scintillation layer 20, e.g., of cesium iodide (CsI), absorbs and converts impinging X-ray photons to visible light photons for detection by photosensitive elements within the detector array 22, e.g., of amorphous silicon (a-Si). The thickness of the scintillation layer 20 is selected so as to absorb sufficient X-ray photons and produce sufficient visible photons so as to generate an adequate SNR for fluoroscopic operation. Similarly, the columns, or "needles," of the crystalline CsI are selected so as to have diameters sufficiently small to support the spatial resolution sampling desired for radiographic operation.

The detector array 22 is designed in accordance with well known techniques into a two dimensional array of microscopic squares referred to as picture elements, or "pixels." Each pixel is composed of an addressable photosensitive element, such as a photodiode and switching transistor combination. As discussed in more detail below, each pixel is accessed in accordance with drive signals from off-array driver circuit assemblies 26a, 26b which provide addressing control signals. In accordance with well.known techniques, the lateral dimensions of the photodiodes are made sufficiently small to provide the desired spatial resolution imaging for radiographic operation and the capacitance of the photodiodes is designed to be sufficiently large to provide the desired signal handling capacity for accommodating the largest signal produced during radiographic operation.

The pixel data accessed by the driver circuits 26 are read out by a receiver, or readout, circuit assembly 28, as discussed in more detail below. The receiver circuit assembly 28 and detector array 22 are mounted on opposing sides of a base plate 24. (The receiver circuit assembly 28 is placed beneath the array 22 so as to minimize the lateral size of the detector cassette 12 and thereby make the detector cassette 12 approximately the same size as a film cassette. If so desired, the driver circuits 26 can also be placed beneath the array 22.)

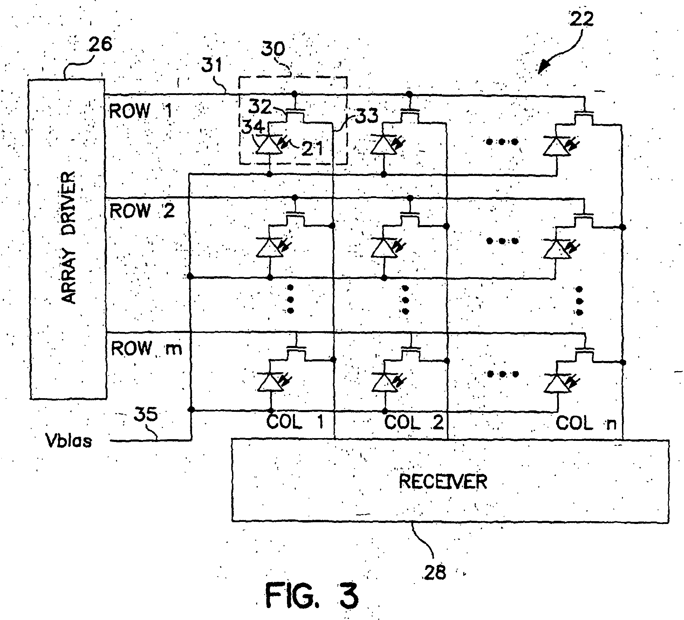

Referring to Figure 3, the detector array 22, as noted above, is composed of a two dimensional array, or matrix, of photosensitive pixels 30 which, in a preferred embodiment, include a switching transistor 32 and a photodiode 34. The anode of the photodiode 34 is biased by a biasing voltage 35 to establish a capacitance for storing electrical charges which accumulate due to the reception of incident light 21 from the scintillation layer 20 (Figure 2). When the pixel 30 is accessed, a row address signal 31 from the array driver circuit 26 (discussed in more detail below) drives the gate of the switching transistor 32 (TFT), thereby providing a column data signal 33 representing the stored charge from the photodiode 34. This signal 33 is received and buffered by a charge sensitive amplifier within the receiver circuit assembly 28 (discussed in more detail below).

Each row address signal 31 is asserted for a predetermined period of time, referred to as "line time." During assertion of each row address signal 31, the signal 33 from each pixel along that row is transmitted via the column data lines to the receiver circuit assembly 28 where the signal 33 on each data line is received and buffered by a corresponding charge sensitive amplifier (discussed in more detail below). Hence, an entire row of image data is captured in one line time period. With each subsequent line time period, a subsequent row of image data is captured. At the end of a "frame time" period, the entire image has been captured. In this manner, each pixel contained in the entire active detection area is sampled individually.

Based upon the foregoing, and in accordance with the more detailed discussions of the driver 26 and receiver 28 circuit assemblies which follow, it can be seen that the pixel array supports multiple modes of operation. For example, during radiographic operation, the pixel data is sampled on a pixel-by-pixel basis as discussed above. However, during fluoroscopic operation, pixel data access can be accelerated, albeit with a reduction in spatial resolution. This can be done by combining, or "binning," multiple pixels to produce "super pixels." For example, a two-by-two pixel subset in which two rows and columns of pixels are combined can be created by addressing two adjacent rows and two adjacent columns of pixels at one time, with the driver circuit assembly 26 performing the simultaneous row addressing and the receiver circuit assembly 28 performing the column line signal combining. Hence, while the spatial resolution is reduced accordingly, significantly less time will be required to capture the image, thereby allowing fluoroscopic imaging-to be performed.

This use of super pixels can also be done in a more selective manner. For example, image acquisition in a fluoroscopic magnification mode can be performed when only a portion of the active detection area is of interest. During such operation, the rows outside the region of interest are addressed at a rapid rate or skipped entirely, while the rows within the region of interest are addressed at a slower rate. The overall time to sequence through or skip past all of the rows, i.e., the frame time, can remain equal to the frame time associated with the fluoroscopic normal mode. However, due to the increased time available within the region of interest, the super pixels within such region can be reduced in size, thereby increasing the spatial resolution. (Appropriate combining of column line signals is also used accordingly.) Hence, the smaller the size of the super pixel in the region of interest, the higher the apparent magnification. (A smaller area of the detector is captured when operating in fluoroscopic magnification mode than when operating in fluoroscopic normal mode, but the display area remains the same, thereby producing an apparent magnification.)

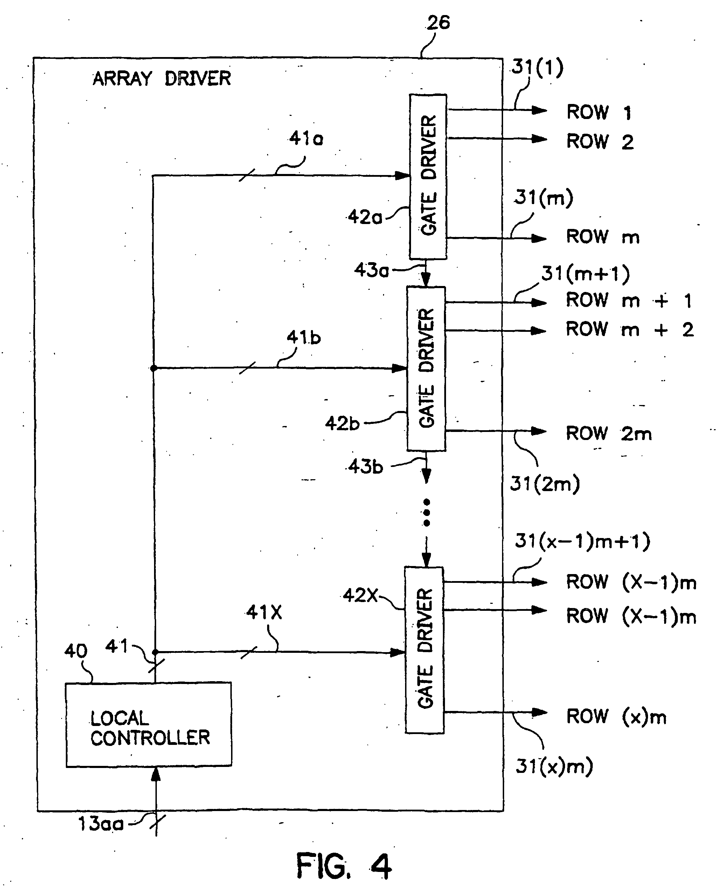

Referring to Figure 4, the driver circuit assembly 26 includes a local controller 40 for receiving control signals 13aa from the computer and control system 14 (Figure 1), plus a series of gate drivers 42 for providing the row addressing signals 31. These gate drivers 42 can be operated in the manner of shift registers or, alternatively, be individually programmed as desired according to the mode of operation using the control signals 41 from the local controller 40. For example, during radiographic operation, the driver circuits 42 can be programmed such that the row 1 addressing signal 31(1) is asserted while the remaining row addressing signals are de-asserted. Immediately following the next line synchronization cycle, the row 1 signal is de-asserted and the row 2 signal is asserted, while the remaining row signals are de-asserted. This successive assertion and de-assertion of signals is repeated until all rows have been addressed. During fluoroscopic operation, the foregoing assertion and de-assertion sequence is repeated, with the exception that multiple adjacent row address signals are asserted at one time for creating super pixels, as discussed above.

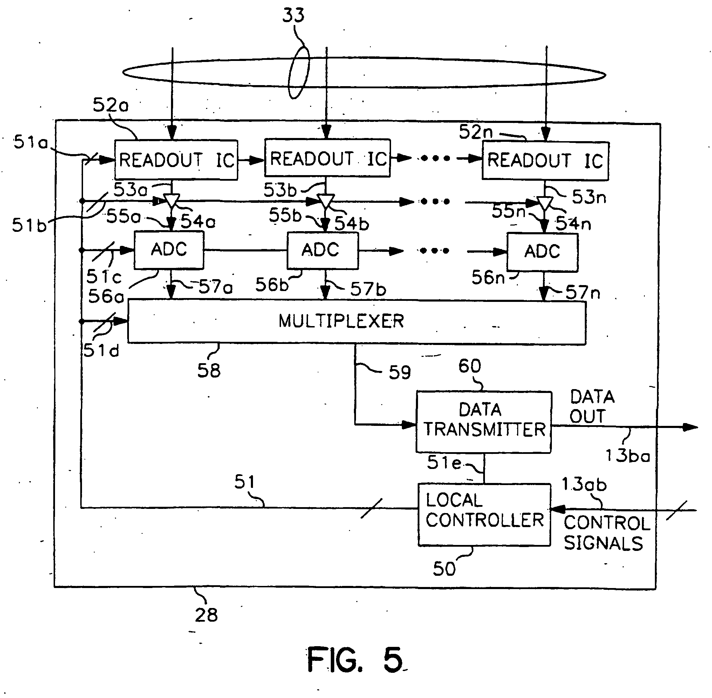

Referring to Figure 5, the receiver circuit assembly 28 includes a local controller 50 for receiving control signals 13ab from the computer and control system 14 (Figure 1) and generating local control signals 51. In accordance with its local control signals 51a, a number of readout circuits 52 (discussed in more detail below), the number of which depends upon the number of columns to be read out from the detector array 22, receives the column data signals 33. The outputs 53 from the readout circuits 52 are buffered by respective transimpedance amplifiers 54. These transimpedance amplifiers 54 are controlled by local control signals 51b for purposes of controlling their offset and gain characteristics (discussed in more detail below). The buffered column data signals 55 are converted by analog-to-digital converters (ADCs) 56. The resulting digitized column data signals 57 are then multiplexed by a multiplexor. The resulting multiplexed data signals 59 are buffered by a data transmitter 60 for transmission to the computer and control system 14.

The control signals 51b for-the transimpedance amplifiers 54 are used to selectively optimize the offset and gain characteristics of the amplifiers 54. This allows the amplifiers 54 to be biased to match the respective output signal ranges of the amplifiers 54 to the input signal ranges of the corresponding ADCs 56.

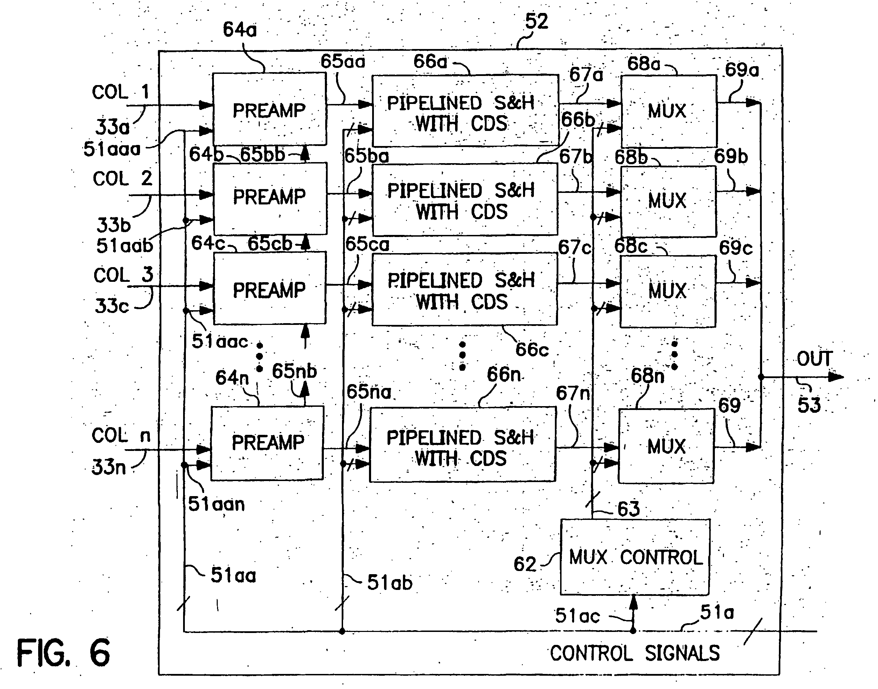

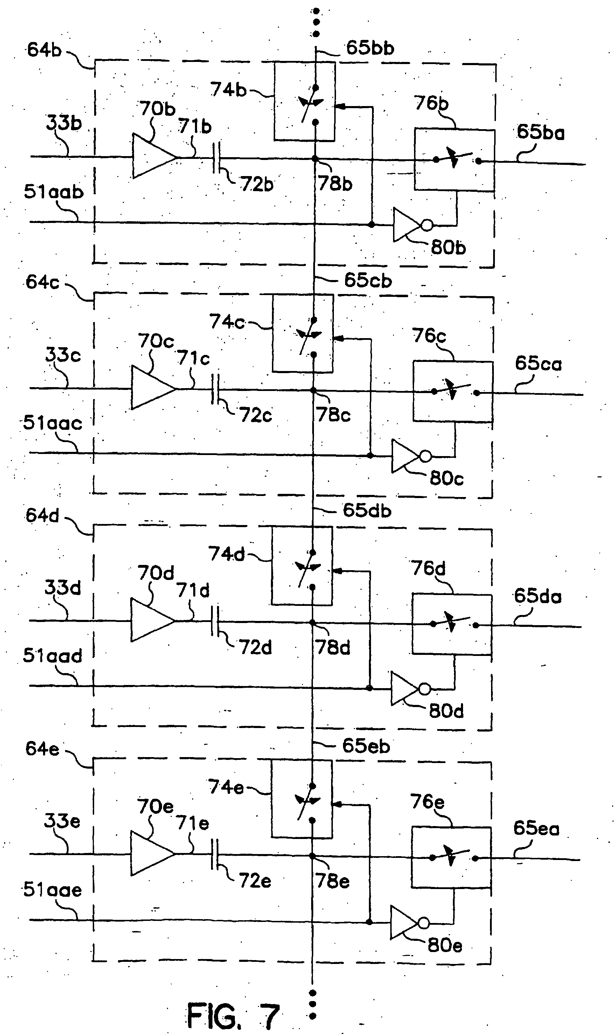

Referring to Figure 6, the readout circuits 52 collectively include multiple input preamplifiers 64, pipelined sample and hold circuits 66 and output multiplexors 68, interconnected substantially as shown. The control signals 51a from the local controller 50 (Figure 5) control the preamplifiers 64, pipelined sample and hold circuits 66 and a multiplexor controller 62 which, in turn, controls the multiplexors 68 via multiplexor control signals 63. The preamplifiers 64 receive the column data signals 33 with charge sensitive amplifiers and provide the aforementioned binning capability for creating super pixels (in conjunction with the multiple row addressing capability of the array driver circuit 26 (Figure 4) as discussed above). The charge sensitive amplifiers are discussed in more detail in copending, commonly assigned U.S. patent application no. 08/758,538, entitled "Charge Sensitive Amplifier With High Common Mode Signal Rejection," filed November 29, 1996. (The pixel binning capability provided by the preamplifiers is discussed in more detail below in connection with Figure 7.)

The buffered output signals 65aa, 65ba, 65ca, from the preamplifiers 64, are sampled using correlated double sampling by the pipelined sample and hold circuits 66 in accordance with their respective control signals 51ab. These pipelined sample and hold circuits 66 are described in more detail in copending, commonly assigned U.S. patent application no. 08/758,536, entitled "Pipelined Sample and Hold Circuit With Correlated Double Sampling," filed November 29, 1996.

The sampled data signals 67 are multiplexed by their respective multiplexors 68 to provide the final output signal 53. These multiplexors 68 operate in an analog current mode and are described in more detail in copending, commonly assigned U.S. patent application no. 08/758,528, entitled "Current Mode Analog Signal Multiplexor," November 29, 1996.

Referring to Figure 7, the aforementioned pixel binning capability with respect to the column data can be described as follows. For purposes of this explanation, the second 64b, third 64c and fourth 64d preamplifier circuits are illustrated to represent the interconnection among adjacent preamplifiers 64. Internal to each preamplifier 64 is the aforementioned charge sensitive amplifier 70 which receives the column data signal 33. The buffered column data signal 71 is coupled by a series coupling capacitor 72 to a summing node 78 for selectively being summed with the buffered and capacitively coupled column data signal from its adjacent preamplifier circuit 64. For example, if one-by-two super pixels were being used, then the third and fourth pixels would be binned together by appropriately asserting and de-asserting the control signals in signal sets 51aac and 51aad (and their inverse equivalents via inverters 80c and 80d) so that switches 74c, 74e and 76d are opened and switches 74d and 76c are closed. Accordingly, the buffered and capacitively coupled data signal 65db from the fourth preamplifier 64d is summed with that of the third preamplifier 64c at its summing mode 78c for outputting as binned pixel data signal 65ca.

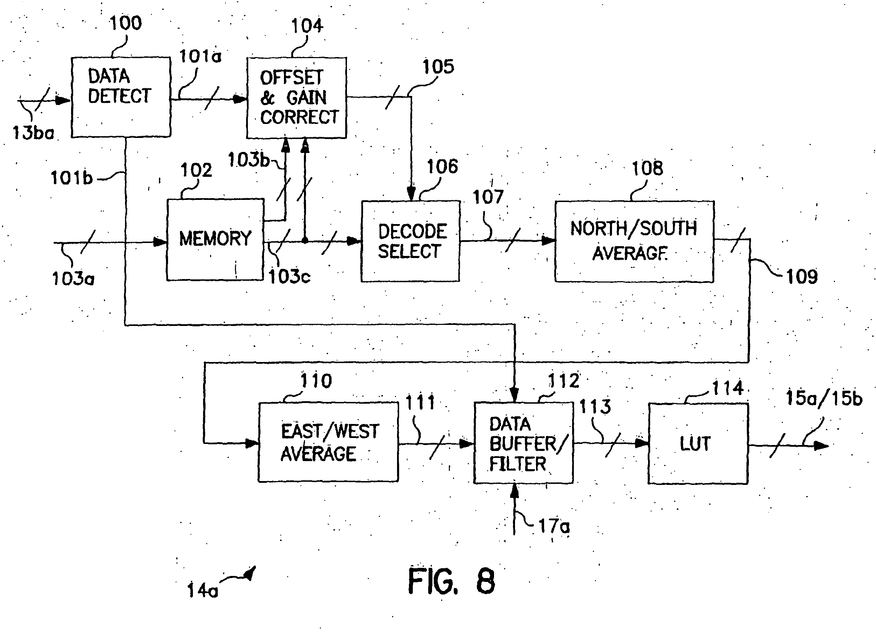

Referring to Figure 8, that portion 14a of the computer and control system 14 (Figure 1) responsible for processing the image data for display in accordance with the selected mode of operation functions as follows. The sampled image data 13ba from the detector cassette 12 (Figure 1) is detected by a data detection stage 100. The data detection stage 100 monitors selected portions of the frame of incoming data and, in addition to passing the incoming data 101a on to the next stage, generates a data present status signal 101b for use elsewhere within the system (as discussed in more detail below).

The buffered sampled image data 101a is corrected by an offset-and gain correction stage 104 using offset and gain correction data 103b, 103c stored in a memory 102. Such offset and gain correction data 103b, 103c can be acquired in accordance with well known techniques. For example, the offset correction data, used to correct for the effects of leakage currents within the detector array 22 (Figure 2), can be collected by processing dark frames of pixel data (no X-ray photons received) and inputted as a portion of pixel mapped data 103a to the memory 102. The gain correction data, used for normalizing the gain profile of the detector array 22, can be collected by processing frames of pixel data generated when receiving an unobstructed X-ray field, and inputted as a portion of pixel mapped data 103a to the memory 102. Such data is used to correct for variations in pixel quantum efficiency and the two dimensional gain profile of the detector array 22 together with its associated receiver circuitry 28.

In addition to the offset and gain information, one of the correction data words 103c (e.g., corresponding to that normally used for gain correction data) also includes pixel flag data and pixel data averaging instruction bits which identify, on a pixel-by-pixel basis, which pixels, if any, are defective and the nature of such defect and, therefore, require the use of either north/south or east/west averaging. Such pixel flag data can be collected in accordance with well known techniques. For example, defective pixels can be identified while collecting the offset and/or gain correction data.

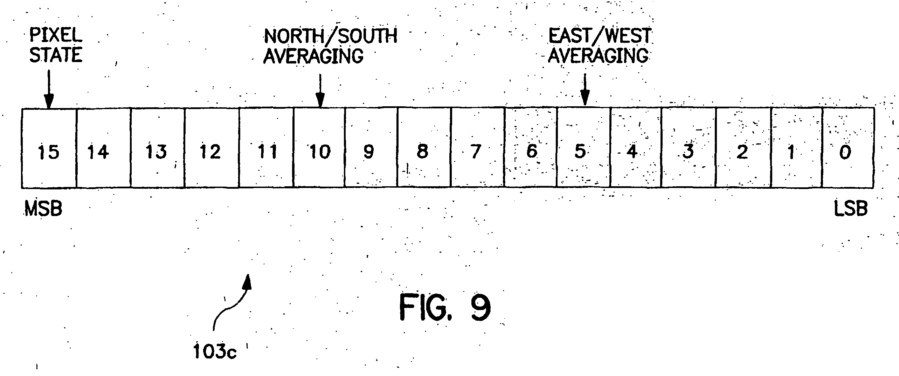

Referring to Figure 9, the format of the correction data word 103c corresponding to a bad pixel is as shown. (It should be understood that other selected bits within the word 103c can be used, as desired). The most significant bit is used to identify the state of the pixel, i.e., whether the pixel represents valid data or is defective and, therefore, represents invalid data. Other bits, e.g., bits 10 and 5, are used to indicate whether north/south or east/west averaging is to be performed. When the pixel data flag identifies the pixel as containing valid data, the remaining bits contain the actual offset or gain correction information to be used by the offset and gain correction stage 104.

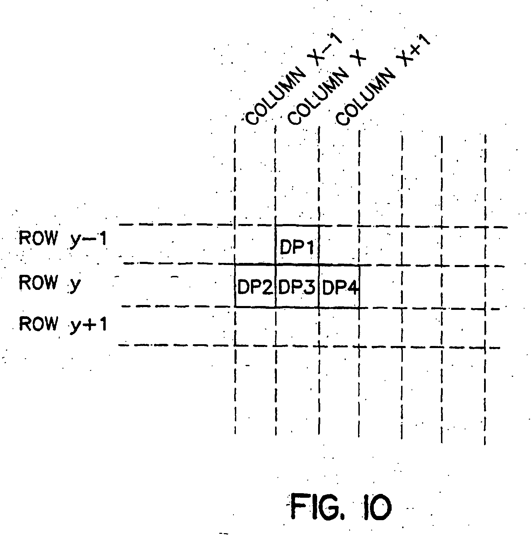

Referring to Figure 10, the decision criteria for determining whether north/south or east/west pixel data averaging is to be performed can be explained as follows. In this example, a group of four defective pixels DP1-DP4 have been identified in columns X-1 through X+1 and rows Y-1 and Y, as shown. For this configuration of defective pixels, the data correction words 103c corresponding to these defective pixels, in addition to containing asserted pixel flags identifying these pixels as being defective, will contain asserted north/south and east/west averaging instruction bits. For defective pixels DP2 and DP4, a north/south averaging will be performed using the pixel data immediately above and below in columns X-1 and X+1. Since this portion of columns X-1 and X+1 contain "good" pixel data, the defective pixels DP1 and DP3 will be processed using east/west averaging. It is preferred that an east/west average be followed by a north/south and thereafter followed by a second east/west average to allow different arrangements of defective pixels to be corrected.

Referring again to Figure 8, the offset and gain correction stage 104 processes the incoming data 101a on a pixel-by-pixel basis in accordance with the correction data 103b, 103c regardless of whether any such data is identified as originating from a bad pixel. The resulting corrected data 105 is then provided, along with the one correction data word 103c to a decode/select circuit 106. If, according to the pixel flag data within the correction data word 103c from the memory 102, the corrected pixel data 105 did not originate from a defective pixel, the decode/select circuit 106 provides the corrected pixel data 105 as its output signal 107. If, however, the pixel flag data identify the "corrected data" 105 as having originated from a defective pixel, the decode/select circuit 106 provides the correction data 103c from the memory 102 as its output signal 107 for dynamic processing along with other valid corrected pixel data by the remainder of this processing section 14a.

The data 107 from the decode/select stage 106 is then processed, in accordance with the north/south and east/west averaging instruction bits discussed above, by a north/south averaging circuit 108 or an east/west averaging circuit 110. For example, if the selected data 107 contains an instruction bit identifying north/south averaging as being required, the north/south averaging stage 108 produces appropriately averaged data 109 which is then simply passed through the east/west averaging stage 110 without further processing. However, if the selected data 107 contains an instruction bit identifying east/west averaging as being required, the selected data 107 is simply passed through the north/south averaging stage 108 and presented to the east/west averaging stage 110 for appropriate processing therein.

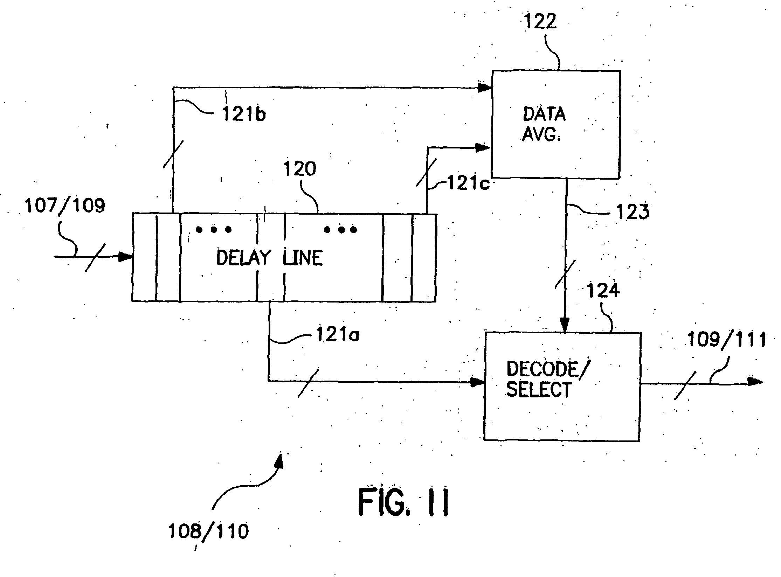

Referring to Figure 11, the averaging function performed by the north/south 108 and east/west 110 averaging stages can be described as follows. The incoming data 107/109 is inputted continuously to a delay line 120 (e.g., a shift register). For the north/south averaging stage 108, this delay line 120 is large enough to contain slightly more than two rows of pixel data, while for the east/west averaging stage 110, the delay line 120 need only be large enough to accommodate three or more pixels worth of data. When a data word 107/109 containing an asserted averaging instruction bit which corresponds to that particular averaging stage 108/110 is encountered, it is provided as an output signal 121a to a decode/select stage 124 when it has reached the approximate midpoint of the delay line 120.

Upon receiving this signal 121a, the decode/select stage 124, recognizing the instruction, uses the averaged pixel data 123 provided by a data averaging stage 122. (The data averaging operation performed by this stage 122 can be done in accordance with any of several well known techniques, e.g., interpolation of selected adjacent pixel data.) If, however, this midpoint signal 121a from the delay line 120 does not contain an asserted averaging instruction bit, but, instead, simply contains normal pixel data, the decode/select stage 124 will ignore the averaged pixel data 123 constantly being provided by the data averaging stage 122 and simply provide this pixel data signal 121a as its output data.signal 109/111.

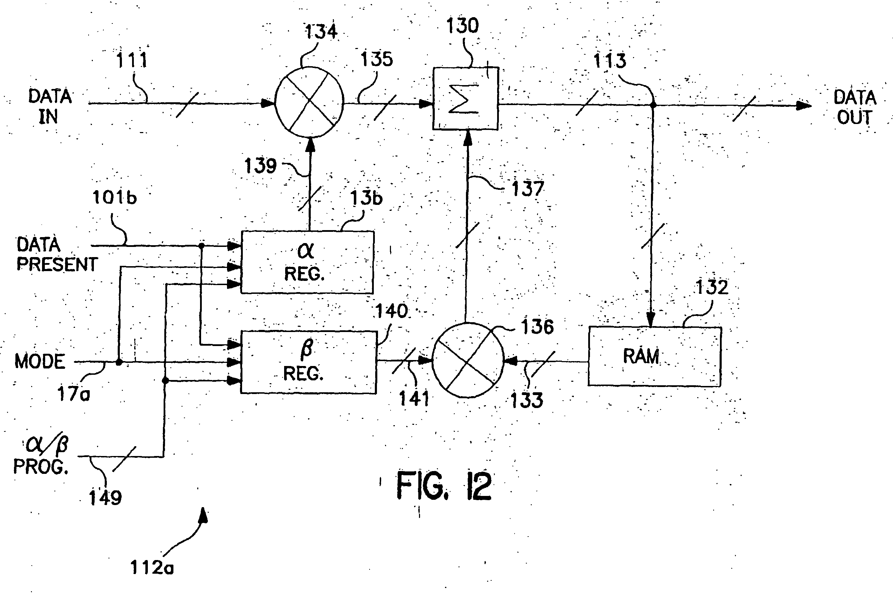

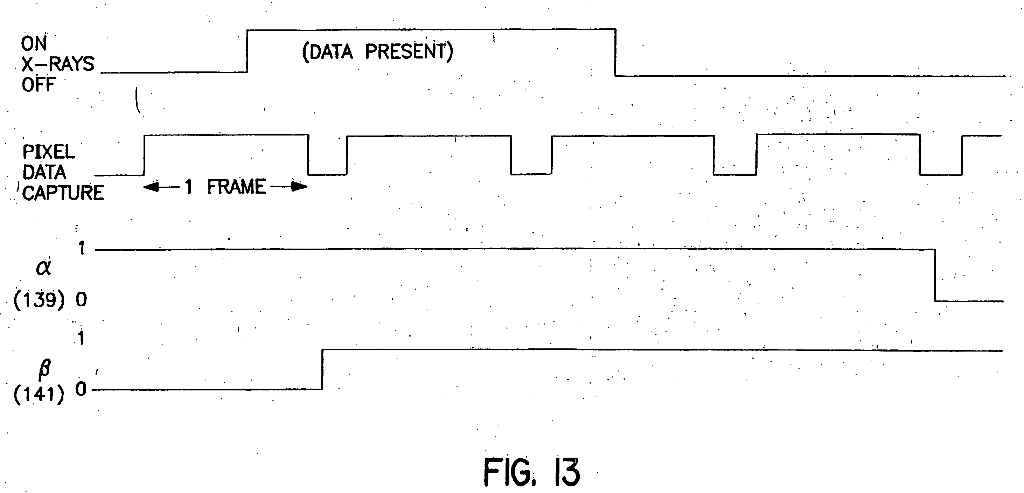

Referring again to Figure 8, the final averaged pixel data 111 is then selectively processed by a data buffer/filter 112. The operation of this data buffer filter 112 for the different modes of operation can be explained as follows with reference to Figures 12 and 13. A preferred embodiment 112a of the data buffer/filter 112 includes an adder 130, a memory (e.g., random access memory) 132, two scaling circuits (e.g., multipliers) 134, 136, and two data registers 138, 140. The memory 132 is used to continuously receive and store the output data 113 on a frame-by-frame basis and provide such stored data in a first-in, first-out manner to one of the scaling circuits 136. The data registers 138, 140 contain data corresponding to scaling factors α and β for scaling the present pixel data 111 and corresponding prior pixel data 133 (i.e., from a previous frame), respectively. The data registers 138, 140 use the aforementioned data present status signal 101b, a mode control signal 17a (originating from within the computer and control system 14) and an α/β programming signal 149 for establishing the values of the α 139 and β 141 data provided to the scaling circuits 134, 136. The mode control signal 17a identifies whether a still or motion image operation is to be performed, while the α/β programming signal 149 can be used to program the actual values for α and β.

When operating in a still image (e.g., radiographic) mode, the α data 139 and β data 141 are initialized at unity and zero, respectively. When the data present status signal 101b indicates that valid active data has begun being received, the β data 141 is switched from zero to unity. Accordingly, scaled data sets 135 and 137 are equal to the present frame pixel data 111 and prior frame pixel data 133, respectively, thereby resulting in frame summation. This summation of prior and present frames of pixel data is done to generate the complete data set for display and is necessary since all data associated with a still image is generally not captured in a single reading of one frame of data, as represented in Figure 13. Once the data present status signal 101b has indicated that valid active data is no longer being received, and after one additional frame of data has been collected, the α data 138 is reset to zero, while the β data 141 remains at unity.

When operating in a motion image (e.g., fluoroscopic) mode, the present data frame scaling factor α 137 is set equal to a predetermined value between zero and unity. Such a value can be established empirically to provide a video display with the desired characteristics. The prior data frame scaling factor β 141 is set at a value which is also between zero and unity and is equal to 1-α. Hence, the output data 113 to be used for display purposes is composed primarily of the present frame of pixel data summed with a small portion of the prior frame of pixel data as stored by the memory 132. This has the effect of slightly increasing the SNR of the displayed frame as compared to the frame as captured by the detector array 22. (Also, it has the additional effect of smoothing out any observable motion within the displayed image).

Referring again to Figure 8, the buffered and/or filtered pixel data 113 is used to address a lookup table 114 for purposes of mapping the input pixel data 113 to pixel data 15a/15b which is appropriately scaled for the particular display device being used. Alternatively, this output stage 114 can include additional circuitry, as desired, to provide actual video signals for directly driving a display monitor.

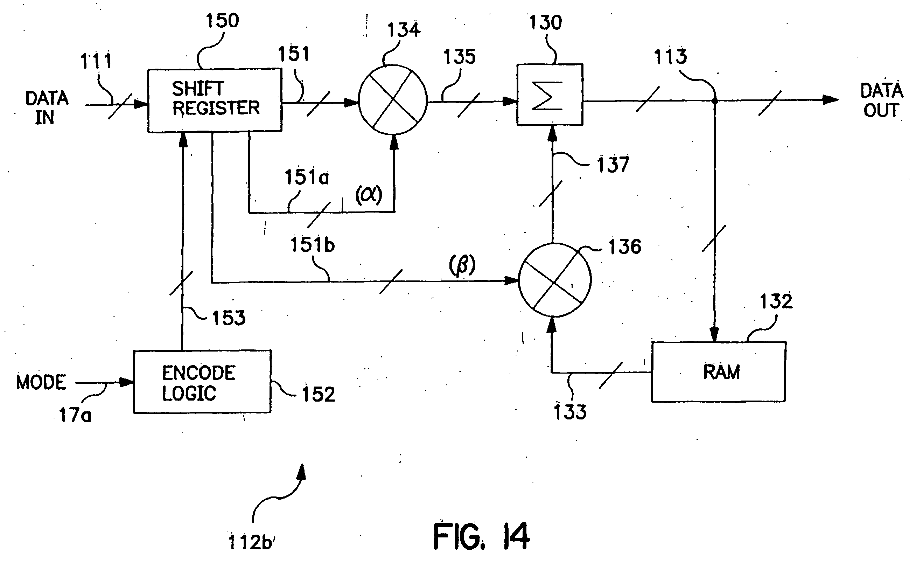

Referring to Figure 14, an alternative embodiment 112b of the data buffer/filter stage 112 can be implemented as shown. (Those elements which correspond to those in the embodiment 112a of Figure 12 are identified with the same numeric designators.) This embodiment 112b can be used where the α and β scaling factor data has been inserted or encoded in some manner within the incoming data stream 111. Accordingly, a shift register 150 can be used to buffer and delay the actual pixel data while the α data 151 a and β data 151b are removed and forwarded on to the scaling circuits 134, 136. An encode logic circuit 152 can be used to provide appropriate control signals 153, in accordance with the mode control signal 17a, to the shift register 150.

Various other modifications and alterations in the structure and method of operation of this invention will be apparent to those skilled in the art without departing from the scope of the invention. Although the invention has been described in connection with specific preferred embodiments, it should be understood that the invention as claimed should not be unduly limited to such specific embodiments. It is intended that the following claims define the scope of the present invention and that structures and methods within the scope of these claims be covered thereby.

## Claims

1. An image acquisition system (10), comprising:
a detector array (22) comprised of a plurality of detectors (30) arranged in a plurality of rows and a plurality of columns and configured to generate a plurality of image signals (33) in response to photons (21) illuminating the plurality of detectors (30);
a first driver circuit (26a) coupled to a first plurality of rows of detectors (30) in said detector array (22) and configured to sequentially generate a plurality of first addressing signals (31), each addressing one or more rows of detectors (30) in the first plurality of rows of detectors (30);
a readout circuit (28) coupled to said detector array (22) and including a plurality of column access units (52), each receiving the image signals (33) from a corresponding column of detectors (30);
a binning circuit coupled to said readout circuit (28) and configured to generate a plurality of column data (65), each in response to the image signals (71) at one or more column access units (52) in said readout circuit;
a data transmitting circuit (60) coupled to said binning circuit and configured to generate a data signal (15) in response to the plurality of column data (65); and
a display device (18) coupled to said data transmitting circuit (60) and configured to generate a visual image in response to the data signal (15).

2. The image acquisition system (10) of claim 1, further comprising a second driver circuit (26b) coupled to a second plurality of detectors (30) in said detector array (22) interleaving with the first plurality of detectors (30) and configured to sequentially generate a plurality of second addressing signals (31), each addressing one or more rows of detectors (30) in the second plurality of rows of detectors (30).

3. The image acquisition system (10) of claim 2, wherein:
each of the plurality of first addressing signals (31) sequentially generated by said first driver circuit (26a) addresses one row of detectors (30) in the first plurality of rows of detectors (30);
each of the plurality of second addressing signals (31) sequentially generated by said second driver circuit (26b) addresses one row of detectors (30) in the second plurality of rows of detectors (30); and
said binning circuit generates each of the plurality of column data (65) by combining the image signals (33) at one column access unit.

4. The image acquisition system (10) of claim 1, wherein:
each of the plurality of first addressing signals (31) sequentially generated by said first driver circuit (26a) addresses a first integral number of rows of detectors (30) in response to a first control signal (41) and addresses a second integral number of rows of detectors (30) in response to a second control signal (41), the second integral number being different from the first integral number; and
said binning circuit generates each of the plurality of column data (65) by combining the image signals (33) at a first number of column access units (52) in response to the first control signal (51) and generates each of the plurality of column data (65) by combining the image signals (33) at a second number of column access units (52) in response to the second control signal (51).

5. The image acquisition system (10) of claim 4, wherein:
the first integral number is one;
the first number is one; and
said display device (18) generates the visual image in a still image mode in response to the first control signal and generates the visual image in a motion image mode in response to the second control signal.

6. The image acquisition system (10) of claim 1, further comprising a data processing circuit (104) and a data selection circuit (106) serially coupled between said data transmitting circuit (60) and said display device (18).

7. The image acquisition system (10) of claim 6, further comprising a data scaling and summing circuit (130) and a data memory unit (132) coupled between said data selection circuit (106) and said display device (18).

8. The image acquisition system (10) of claim 1, wherein each of the plurality of detectors (30) in said detector array (22) includes:
a photo sensitive device (34) having a first electrode coupled for receiving a bias voltage (35) and a second electrode; and
a switch (32) having a control electrode coupled to said first driver circuit (26), a first conducting electrode coupled to the second electrode of said photo sensitive device (34), and a second conducting electrode coupled to said first readout circuit (28).

9. The image acquisition system (10) of claim 1, wherein said binning circuit includes a plurality of binning elements (64), each coupled to a corresponding column access unit (52) in said first readout circuit (28) and comprising:
a column combining switch (74) having a control electrode coupled for receiving a corresponding binning signal (51), a first conducting electrode coupled to the corresponding column access unit (52), and a second conducting electrode coupled to the first conducting electrode of a column combining switch (74) in an adjacent binning element (64);
an inverter (80) having an input terminal coupled to the control electrode of said column combing switch (74) and an output terminal; and
a transmission switch (76) having a control electrode coupled the output terminal of said inverter (80), a first conducting electrode coupled to the first conducting electrode of said column combining switch (74), and a second conducting electrode coupled to said first data transmitting circuit (60).

10. The image acquisition system (10) of claim 1, wherein the plurality of detectors (30) in said detector array (22) generate the plurality of image signals (33) in response to X-ray photons (21) illuminating thereon.

11. A method for forming an image of an object, comprising the steps of:
illuminating a detector array (20) having a plurality of detectors (30) arranged in a plurality of rows and a plurality of columns with photons emitted from a radiation source while placing the object between the radiation source and the detector array (20);
generating a plurality of image signals (33) at the plurality of detectors (30) in response to the photons (21) illuminating thereon;
sequentially reading the image signals (33) by rows in a plurality of readout actions, each readout action reading the image signals from a first integral number of rows of detectors (30) in response to a first control signal (41) and reading the image signals from a second integral number of rows of detectors (30) in response to a second control signal (41), the second integral number being different from the first integral number;
generating an array of binned image signals (65) by binning the image signals (33) from a first number of columns of detectors (30) in response to the first control signal (51) and binning the image signals (33) from a second number of columns of detectors (30) in response to the second control signal (51); and
generating an image of the object using the array of binned image signals (65).

12. The method as claimed in claim 11, wherein the step of sequentially reading the image signals (33) by rows in a plurality of readout actions includes the step of sequentially generating a plurality of row activation signals (31), each activating the detectors (30) in the corresponding first integral number of rows in response to the first control signal (41) and activating the detectors (30) in the second integral number of rows in response to the second control signal (41).

13. The method as claimed in claim 11, further comprising the steps of:
substituting a binned image signal in the array of binned image signals corresponding to a first row address and a first column address with an average of two binned signals in the array of binned image signals corresponding to two row addresses adjacent to the first row address and the first column address in response to a row averaging signal; and
substituting a binned image signal in the array of binned image signals corresponding to the first row address and the first column address with an average of two binned signals in the array of binned image signals corresponding to the first row address and two column addresses adjacent to the first row address in response to a column averaging signal.

14. The method as claimed in claim 11, further comprising the steps of:
detecting a start of a period for the photons illuminating the detector array;
multiplying the array of binned image signals generated during the period by a first factor to generate a present image signal in response to the start of the period;
multiplying an array of summed image signals stored in a buffer by a second factor to generate a previous image signal;
summing the present image signal and the previous image signal to regenerate the array of summed image signals;
storing the array of summed image signals in the buffer;
detecting an end of the period; and
adjusting the first factor to zero and the second factor to one in response to the end of the period.

15. The method as claimed in claim 14, wherein the step of generating an image of the object using the array of binned image signals includes outputting the array of summed image signals to generate the image of the object.

## Patentansprüche

1. Bilderfassungssystem (10), das umfasst:
eine Detektoranordnung (22), die aus einer Vielzahl von Detektoren (30) besteht, die in einer Vielzahl von Reihen und einer Vielzahl von Spalten angeordnet und so ausgeführt sind, dass sie in Reaktion auf Photonen (21), die die Vielzahl von Detektoren (30) beleuchten, eine Vielzahl von Bildsignalen (33) erzeugen;
eine erste Treiberschaltung (26a), die mit einer ersten Vielzahl von Reihen von Detektoren (30) in der Detektoranordnung (22) gekoppelt und so ausgeführt ist, dass sie sequentiell eine Vielzahl erster Adressierungssignale (31) erzeugt, die eine oder mehrere Reihen von Detektoren (30) in der ersten Vielzahl von Reihen von Detektoren (30) adressieren;
eine Ausleseschaltung (28), die mit der Detektoranordnung (22) gekoppelt ist und eine Vielzahl von Spaltenzugriffseinheiten (52) enthält, die jeweils die Bildsignale (33) von einer entsprechenden Spalte von Detektoren (30) empfangen;
eine Binning-Schaltung, die mit der Ausleseschaltung (28) gekoppelt und so ausgeführt ist, dass sie jeweils in Reaktion auf die Bildsignale (71) an einer oder mehreren Spaltenzugriffseinheiten (52) in der Ausleseschaltung eine Vielzahl von Spaltendaten (65) erzeugt;
eine Datenübertragungsschaltung (60), die mit der Binning-Schaltung gekoppelt und so ausgeführt ist, dass sie in Reaktion auf die Vielzahl von Spaltendaten (65) ein Datensignal (15) erzeugt; und
eine Anzeigevorrichtung (18), die mit der Datenübertragungsschaltung (60) gekoppelt und so ausgeführt ist, dass sie in Reaktion auf das Datensignal (15) ein sichtbares Bild erzeugt.

2. Bilderfassungssystem (10) nach Anspruch 1, das des Weiteren eine zweite Treiberschaltung (26b) umfasst, die mit einer zweiten Vielzahl von Detektoren (30) in der Detektoranordnung (22) gekoppelt ist, die mit der ersten Vielzahl von Detektoren (30) verschachtelt sind, und die so ausgeführt ist, dass sie sequentiell eine Vielzahl zweiter Adressiersignale (31) erzeugt, die jeweils eine oder mehrere Reihen von Detektoren (30) in der zweiten Vielzahl von Reihen von Detektoren (30) adressieren.

3. Bilderfassungssystem (10) nach Anspruch 2, wobei:
jedes der Vielzahl erster Adressiersignale (31), die sequentiell durch die erste Treiberschaltung (26a) erzeugt wurde, eine Reihe von Detektoren (30) in der ersten Vielzahl von Reihen von Detektoren (30) adressiert;
jedes der Vielzahl zweiter Adressiersignale (31), die sequentiell durch die zweite Treiberschaltung (26b) erzeugt wurde, eine Reihe von Detektoren (30) in der zweiten Vielzahl von Reihen von Detektoren (30) adressiert; und
die Binning-Schaltung alle der Vielzahl von Spaltendaten (65) durch Kombinieren der Bildsignale (33) an einer Spaltenzugriffseinheit erzeugt.

4. Bilderfassungssystem (10) nach Anspruch 1, wobei:
jedes der Vielzahl erster Adressiersignale (31), die sequentiell durch die erste Treiberschaltung (26a) erzeugt wurde, in Reaktion auf ein erstes Steuersignal (41) eine erste ganze Zahl von Reihen von Detektoren (30) adressiert und in Reaktion auf ein zweites Steuersignal (42) eine zweite ganze Zahl von Reihen von Detektoren (30) adressiert, wobei sich die zweite ganze Zahl von der ersten ganzen Zahl unterscheidet; und
die Binning-Schaltung alle der Vielzahl von Spaltendaten (65) durch Kombinieren der Bildsignale (33) an einer ersten Anzahl von Spaltenzugriffseinheiten (52) in Reaktion auf das erste Steuersignal (51) erzeugt und alle der Vielzahl von Spaltendaten (65) durch Kombinieren der Bildsignale (33) an einer zweiten Anzahl von Spaltenzugriffseinheiten (52) in Reaktion auf das zweite Steuersignal (51) erzeugt.

5. Bilderfassungssystem (10) nach Anspruch 4, wobei:
die erste ganze Zahl Eins ist;
die erste Anzahl Eins ist; und
die Anzeigevorrichtung (18) in Reaktion auf das erste Steuersignal das sichtbare Bild in einem Standbild-Modus erzeugt und in Reaktion auf das zweite Steuersignal das sichtbare Bild in einem Bewegtbild-Modus erzeugt.

6. Bilderfassungssystem (10) nach Anspruch 1, das des Weiteren eine Datenverarbeitungsschaltung (104) und eine Datenauswählschaltung (106) umfasst, die in Reihe zwischen die Datenübertragungsschaltung (60) und die Anzeigevorrichtung (18) gekoppelt sind.

7. Bilderfassungssystem (10) nach Anspruch 6, das des Weiteren eine Daten-Skalier-und-Addier-Schaltung (130) und eine Datenspeichereinheit (132) umfasst, die zwischen die Datenauswählschaltung (106) und die Anzeigevorrichtung (18) gekoppelt sind.

8. Bilderfassungssystem (10) nach Anspruch 1, wobei jeder der Vielzahl von Detektoren (30) in der Detektoranordnung (22) enthält:
eine lichtempfindliche Vorrichtung (34) mit einer ersten Elektrode, die zum Empfangen einer Vorspannung (35) gekoppelt ist, und einer zweiten Elektrode; und
einen Schalter (32) mit einer Steuerelektrode, die mit der ersten Treiberschaltung (26) gekoppelt ist, einer ersten leitenden Elektrode, die mit der zweiten Elektrode der lichtempfindlichen Vorrichtung (34) gekoppelt ist, und einer zweiten leitenden Elektrode, die mit der ersten Ausleseschaltung (28) gekoppelt ist.

9. Bilderfassungssystem (10) nach Anspruch 1, wobei die Binning-Schaltung eine Vielzahl von Binning-Elementen (64) enthält, die jeweils mit einer entsprechenden Spaltenzugriffseinheit (52) in der ersten Ausleseschaltung (28) gekoppelt sind und umfassen:
einen Spaltenkombinier-Schalter (74) mit einer Steuerelektrode, die zum Empfangen eines entsprechenden Binning-Signals (51) gekoppelt ist, einer ersten leitenden Elektrode, die mit der entsprechenden Spaltenzugriffseinheit (52) gekoppelt ist, und einer zweiten leitenden Elektrode, die mit der ersten leitenden Elektrode eines Spalten-Kombinierschalters (74) in einem angrenzenden Binning-Element (64) gekoppelt ist;
einen Inverter (80) mit einem Eingangsanschluss, der mit der Steuerelektrode des Spalten-Kombinierschalters (74) gekoppelt ist, und einem Ausgangsanschluss; und
einem Übertragungsschalter (76) mit einer Steuerelektrode, die mit dem Ausgangsanschluss des Inverters (80) gekoppelt ist, einer ersten leitenden Elektrode, die mit der ersten leitenden Elektrode des Spaltenkombinier-Schalters (74) gekoppelt ist, und einer zweiten leitenden Elektrode, die mit der ersten Datenübertragungsschaltung (60) gekoppelt ist.

10. Bilderfassungssystem (10) nach Anspruch 1, wobei die Vielzahl von Detektoren (30) in der Detektoranordnung (22) die Vielzahl von Bildsignalen (33) in Reaktion darauf erzeugen, dass Röntgenstrahlen-Photonen (21) sie beleuchten.

11. Verfahren zum Ausbilden eines Bildes eines Objektes, das die folgenden Schritte umfasst:
Beleuchten einer Detektoranordnung (20) mit einer Vielzahl von Detektoren (30), die in einer Vielzahl von Reihen und einer Vielzahl von Spalten angeordnet sind, mit Photonen, die von einer Strahlungsquelle emittiert werden, während das Objekt zwischen der Strahlungsquelle und der Detektoranordnung (20) angeordnet wird;
Erzeugen einer Vielzahl von Bildsignalen (33) an der Vielzahl von Detektoren (30) in Reaktion darauf, dass die Photonen (21) diese beleuchten;
sequentielles Lesen der Bildsignale (33) reihenweise in einer Vielzahl von Auslesevorgängen, wobei bei jedem Auslesevorgang in Reaktion auf eine erstes Steuersignal (41) die Bildsignale von einer ersten ganzen Zahl von Reihen von Detektoren (30) gelesen werden und in Reaktion auf ein zweites Steuersignal (41) die Bildsignale von einer zweiten ganzen Zahl von Reihen von Detektoren (30) gelesen werden, wobei sich die zweite ganze Zahl von der ersten ganzen Zahl unterscheidet;
Erzeugen einer Anordnung von Binning unterzogenen Bildsignalen (60) durch Ausführen von Binning der Bildsignale (33) von einer ersten Anzahl von Spalten von Detektoren (30) in Reaktion auf das erste Steuersignal (51) und Binning der Bildsignale (33) von einer zweiten Zahl von Spalten von Detektoren (30) in Reaktion auf das zweite Steuersigna! (51); und
Erzeugen eines Bildes des Objektes unter Verwendung der Anordnung Binning unterzogener Bildsignale (65).

12. Verfahren nach Anspruch 11, wobei der Schritt des sequentiellen, Lesens der Bildsignale (33) in Reihen in einer Vielzahl von Auslesevorgängen den Schritt des sequentiellen Erzeugens einer Vielzahl von Reihenaktivierungs-Signalen (31) enthält, die in Reaktion auf das erste Steuersignal (41) jeweils die Detektoren (30) in der entsprechenden ersten ganzen Zahl von Reihen aktivieren und in Reaktion auf das zweite Steuersignal (41) die Detektoren (30) in der zweiten ganzen Zahl von Reihen aktivieren.

13. Verfahren nach Anspruch 11, das des Weiteren die folgenden Schritte umfasst:
Ersetzen eines Binning unterzogenen Bildsignals in der Anordnung Binning unterzogener Bildsignale, das einer ersten Reihenadresse und einer ersten Spaltenadresse entspricht, durch einen Mittelwert zweier Binning unterzogener Signale in der Anordnung Binning unterzogener Bildsignale, die zwei Reihenadressen entsprechen, die an die erste Reihenadresse und die erste Spaltenadresse angrenzen, in Reaktion auf ein Reihen-Mittlungssignal; und
Ersetzen eines Binning unterzogenen Bildsignals in der Anordnung Binning unterzogener Bildsignale, das der ersten Reihenadresse und der ersten Spaltenadresse entspricht, durch einen Mittelwert zweier Binning unterzogener Signale in der Anordnung Binning unterzogener Bildsignale, die der ersten Reihenadresse und zwei Spaltenadressen entsprechen, die an die erste Reihenadresse angrenzen, in Reaktion auf ein Spalten-Mittlungssignal.

14. Verfahren nach Anspruch 11, das des Weiteren die folgenden Schritte umfasst:
Erfassen eines Beginns eines Zeitraums der Beleuchtung der Detektoranordnung durch die Photonen;
Multiplizieren der während des Zeitraums erzeugten Anordnung Binning unterzogener Signale mit einem ersten Faktor, um in Reaktion auf den Anfang des Zeitraums ein Signal des momentanen Bildes zu erzeugen;
Multiplizieren einer in einem Pufferspeicher gespeicherten Anordnung addierter Bildsignale mit einem zweiten Faktor, um ein Signal des vorangehenden Bildes zu erzeugen;
Addieren des Signals des momentanen Bildes und des Signals des vorangehenden Bildes, um die Anordnung addierter Bildsignale wieder herzustellen;
Speichern der Anordnung addierter Bildsignale in dem Pufferspeicher;
Erfassen eines Endes des Zeitraums; und
Einstellen des ersten Faktors auf Null und des zweiten Faktors auf Eins in Reaktion auf das Ende des Zeitraums.

15. Verfahren nach Anspruch 14, wobei der Schritt des Erzeugens eines Bildes des Objektes unter Verwendung der Anordnung Binning unterzogener Bildsignale das Ausgeben der Anordnung addierter Bildsignale zum Erzeugen des Bildes des Objektes einschließt.

## Revendications

1. Système (10) d'acquisition d'images comprenant :
un réseau de détecteurs (22) comprenant une pluralité de détecteurs (30) disposés sur une pluralité de lignes et une pluralité de colonnes et configurés de manière à produire une pluralité de signaux d'image (33) en réponse à des photons (21) éclairant la pluralité de détecteurs (30);
un premier circuit formant étage d'attaque (26a) couplé à la première pluralité de lignes de détecteurs (30) dans ledit réseau de détecteurs (22) et configuré de manière à générer séquentiellement une pluralité de premiers signaux d'adressage (31), dont chacun adresse une ou plusieurs lignes de détecteurs (30) dans la première pluralité de lignes de détecteurs (30);
un circuit de lecture (28) couplé audit réseau de détecteurs (22) et incluant une pluralité d'unités (52) d'accès aux colonnes, dont chacune reçoit les signaux d'image (33) de la part d'une colonne correspondante de détecteurs (30);
un circuit de combinaison couplé dudit circuit de lecture (28) et configuré de manière à générer une pluralité de données de colonnes (65), chacune en réponse aux signaux d'image (71) dans une ou plusieurs unités d'accès de colonnes (52) dans ledit circuit de lecture;
un circuit de transmission de données (60) couplé audit circuit de combinaison et configuré de manière à générer un signal de données (15) en réponse à la pluralité de données de colonnes (65); et
un dispositif d'affichage (18) couplé audit circuit de transmission de données (60) et configuré de manière à générer une image visuelle en réponse au signal de données (15).

2. Système (10) d'acquisition d'images selon la revendication 1, comprenant en outre un second circuit formant étage d'attaque (26b) couplé à une seconde pluralité de détecteurs (30) dans ledit réseau de détecteurs (22) d'une manière imbriquée avec la pluralité de détecteurs (30) et configurés de manière à définir séquentiellement une pluralité de seconds signaux d'adressage (31), chaque signal adressant une ou plusieurs lignes de détecteurs (30) dans la seconde pluralité de lignes de détecteurs (30).

3. Système (10) d'acquisition d'images selon la revendication 2, dans lequel :
chacun de la pluralité de premiers signaux d'adressage (31) générés séquentiellement par ledit premier circuit formant étage d'attaque (26a) adresse une ligne de détecteurs (30) dans la première pluralité de lignes de détecteurs (30);
chacun de la pluralité de seconds signaux d'adressage (31) générés séquentiellement par ledit second circuit formant étage d'attaque (26b) adresse une ligne de détecteurs (30) dans la seconde pluralité de lignes de détecteurs (30); et
ledit circuit de combinaison génère chacune de la pluralité de données de colonnes (65) par combinaison des signaux d'image (33) dans une unité d'accès de colonnes.

4. Système (10) d'acquisition d'images selon la revendication 1, dans lequel :
chacun de la pluralité de premiers signaux d'adressage (31) générés séquentiellement par ledit premier circuit formant étage d'attaque (26a) adresse un premier nombre entier de lignes de détecteurs (30) en réponse à un premier signal de commande (41) et adresse un second nombre entier de lignes de détecteurs (30) en réponse à un second signal de commande (41), le second nombre entier étant différent du premier nombre entier; et
ledit circuit de combinaison génère chacune de la pluralité de données de colonnes (65) par combinaison des signaux d'image (33) dans un premier nombre d'unités d'accès de colonnes (52) en réponse au premier signal de commande (51) et produit chacune de la pluralité de données de colonnes (65) par combinaison des signaux d'images (33) en un second nombre d'unités d'accès de colonnes (52) en réponse au second signal de commande (51).

5. Système (10) d'acquisition d'images selon la revendication 4, dans lequel :
le premier nombre entier est un;
le premier nombre est un; et
le dispositif d'affichage (18) génère l'image visuelle dans un mode à image fixe en réponse au premier signal de commande et génère l'image visuelle dans un mode d'image animée en réponse au second signal de commande.

6. Système (10) d'acquisition d'images selon la revendication 1, comprenant en outre une unité (104) de traitement de données et un circuit (106) de sélection de données couplés en série entre ledit circuit de transmission de données (60) et ledit dispositif d'affichage (18).

7. Système (10) d'acquisition d'images selon la revendication 6, comprenant en outre un circuit de cadrage d'échelle et de sommation de données (130) et une unité de mémoire de données (132) couplée entre ledit circuit de sélection de données (106) et ledit dispositif d'affichage (18).

8. Système (10) d'acquisition d'images selon la revendication 1, dans lequel la pluralité de détecteurs (30) contenus dans ledit réseau de détecteurs (22) inclut :
un dispositif photosensible (34) possédant une première électrode couplée de manière à recevoir une tension de polarisation (35) et une seconde électrode; et
un interrupteur (32) possédant une électrode de commande couplée audit premier circuit formant étage d'attaque (26), une première électrode conductrice couplée à la seconde électrode dudit dispositif photosensible (34), et une seconde électrode conductrice couplée audit premier circuit de lecture (28).

9. Système (10) d'acquisition d'images selon la revendication 1, dans lequel ledit circuit de combinaison inclut une pluralité d'éléments de combinaison (64), couplés chacun à une unité correspondante d'accès de colonnes (52) dans ledit premier circuit de lecture (28), et comprenant :
un commutateur (74) de combinaison de colonnes, comprenant une électrode de commande couplée de manière à recevoir un signal de combinaison correspondant (51), une première électrode conductrice couplée à l'unité correspondante d'accès de colonnes (52) et une seconde électrode conductrice couplée à la première électrode conductrice d'un commutateur (74) de combinaison de colonnes dans un élément de combinaison adjacent (64);
un inverseur (80) possédant une borne d'entrée couplée à l'électrode de commande dudit commutateur (74) de combinaison de colonnes et une borne de sortie; et
un interrupteur de transmission (76) possédant une électrode de commande couplée à la borne de sortie dudit inverseur (80), une première électrode conductrice couplée à la première électrode conductrice dudit commutateur (74) de combinaison de colonnes, et une seconde électrode conductrice couplée audit premier circuit de transmission de données (60).

10. Système (10) d'acquisition d'images selon la revendication 1, dans lequel la pluralité de détecteurs (30) dudit réseau de détecteurs (22) génèrent la pluralité de signaux d'image (33) en réponse à des photons de rayonnement X (21) éclairant ce réseau de détecteurs.

11. Procédé pour former une image d'un objet, comprenant les étapes consistant à :
éclairer un réseau de détecteurs (20) possédant une pluralité de détecteurs (30) disposés suivant une pluralité de lignes et une pluralité de colonnes, avec des photons émis par une source de rayonnement, en plaçant l'objet entre la source de rayonnement et le réseau de détecteurs (20);
générer une pluralité de signaux d'image (33) dans la pluralité de détecteurs (30) en réponse aux photons (21) éclairant ces derniers;
lire séquentiellement les signaux d'image (33) selon des lignes dans une pluralité d'actions de lecture, chaque action de lecture lisant les signaux d'image provenant d'un premier nombre entier de lignes de détecteurs (30) en réponse à un premier signal de commande (41) et lisant les signaux d'image à partir d'un second nombre entier de lignes de détecteurs (30) en réponse à un second signal de commande (41), le second nombre entier étant différent du premier nombre entier;
générer un réseau de signaux d'image combinés (65) en combinant les signaux d'image (33) à partir d'un premier nombre de colonnes de détecteurs (30) en réponse au premier signal de commande (51) et en combinant les signaux d'images (33) provenant d'un second nombre de colonnes de détecteurs (30) en réponse au second signal de commande (51); et
produire une image de l'objet en utilisant le réseau de signaux d'image combinés (65).

12. Procédé selon la revendication 11, selon lequel l'étape de lecture séquentielle des signaux d'image (33) suivant des lignes dans une pluralité d'actions de lecture inclut l'étape consistant à générer séquentiellement une pluralité de signaux (31) d'activation de lignes, dont chacun active les détecteurs (30) dans le premier nombre entier correspondant de lignes en réponse au premier signal de commande (41) et activant les détecteurs (30) dans le second nombre entier de lignes en réponse au second signal de commande (41).

13. Procédé selon la revendication 11, comprenant en outre les étapes consistant à :
substituer un signal d'image combiné dans le réseau de signaux d'image combinés correspondant à une première adresse de ligne et à une première adresse de colonne avec une moyenne de deux signaux combinés dans le réseau de signaux d'image combinés correspondant à deux adresses de lignes adjacentes à la première adresse de ligne et à la première adresse de colonne en réponse à un signal de formation de la moyenne de lignes; et
substituer un signal d'image combiné dans le réseau de signaux d'image combinés correspondant à la première adresse de ligne et à la première adresse de colonne avec une moyenne de deux signaux combinés dans le réseau de signaux d'image combinés correspondant à la première adresse de ligne et à deux adresses de colonne adjacentes à la première adresse de ligne en réponse à un signal de formation de la moyenne de colonnes.

14. Procédé selon la revendication 11, comprenant en outre les étapes consistant à :
détecter un démarrage d'une période pendant laquelle les photons éclairent le réseau de détecteurs;
multiplier le réseau de signaux d'images combinés générés pendant cette période, par un premier facteur pour générer un signal d'image présent en réponse au démarrage de la période;
multiplier un réseau de signaux d'images additionnés mémorisés par un tampon par un second facteur pour générer un signal d'image précédent;
additionner le présent signal d'image et le signal d'image précédent pour régénérer le réseau de signaux d'image additionnés;
mémoriser le réseau de signaux d'image additionnés dans le tampon;
détecter une fin de la période; et
ajuster le premier facteur sur zéro et le second facteur sur un en réponse à la fin de la période.

15. Procédé selon la revendication 14, selon lequel l'étape de génération d'une image de l'objet en utilisant le réseau de signaux d'image combinés inclut la délivrance du réseau de signaux d'image additionnés pour générer l'image de l'objet.
